# EUROPÄISCHE PATENTANMELDUNG

(11) **EP 0 666 315 A1**
(43) Veröffentlichungstag der Anmeldung: **09.08.1995**
(21) Anmeldenummer: 95100944.8
(22) Anmeldetag: 25.01.1995
(51) Int. Cl.: C12N 9/64, A61K 38/48

(54) **Thrombozytenstabilisierende Faktor-IX-Fragmente, deren Herstellung und Verwendung sowie sie enthaltende Arzneimittel**

(30) Priorität: 02.02.1994 DE 4403057
(71) Anmelder: Möller, Wolfgang, Dipl.-Chem. Dr., 61440 Oberursel (DE); Kotitschke, Ronald Dr., D-63303 Dreieich (DE); Morfeld, Frank Dr., A-1100 Wien (AT)
(72) Erfinder: Möller, Wolfgang, Dipl.-Chem. Dr., 61440 Oberursel (DE); Kotitschke, Ronald Dr., D-63303 Dreieich (DE); Morfeld, Frank Dr., A-1100 Wien (AT)
(74) Vertreter: Meyers, Hans-Wilhelm, Dr.Dipl.-Chem.

(57) **Zusammenfassung**

Fragmente des Blutgerinnungsfaktors IX mit intakter EGF- und Gla-Domäne ohne Faktor IX-Gerinnungsaktivität, chemisch nicht modifiziert und einem Molekulargewicht zwischen ca.12 000 und 50 000 D, erhältlich durch proteolytische Spaltung, als Wirkstoff mit antithrombotischer Wirkung sowie Verfahren zu ihrer Herstellung, ihre Verwendung zur Herstellung von Arzneimitteln und diese Arzneimittel selbst.

## Beschreibung

Die Erfindung betrifft die Fragmente des Blutgerinnungsfaktors IX mit intakter EGF-like- und Gla-Domäne als Wirkstoff zur Stabilisierung der Thrombozyten in vivo.

Ferner betrifft die Erfindung die Herstellung dieses Wirkstoffs, seine Verwendung zur Herstellung von Arzneimitteln sowie diesen Wirkstoff enthaltende Arzneimittel.

Die Hämostase ist ein komplexer Vorgang, an dem eine Reihe von Plasmaproteinen, Blutzellen und Gefäßkomponenten beteiligt sind.
Sie hat die Funktion, über die Gerinnung des Blutes den Organismus vor einem Blutverlust bei Verletzungen des Gefäßsystems zu schützen, und muß gleichzeitig eine Eskalation des Gerinnungsprozesses verhindern, um den Blutstrom im Gesamtorganismus aufrechtzuerhalten. Eine Störung der Hämostase kann zur Bildung von Gefäßverschlüssen, den Thrombosen, führen.

Die Prophylaxe und Behandlung thrombotischer Erkrankungen, wie Herzinfarkt, venösen Thrombosen, DIC und Schlaganfällen, gestaltet sich sehr schwierig, da eine Inhibition der Blutgerinnung auch immer die Gefahr von lebensbedrohenden Blutungen beinhaltet.

Bekannt sind drei Klassen von antithrombotischen Substanzen. Es handelt sich um Thrombininhibitoren, um Vitamin K-Antagonisten und um Thrombozytenaggregationshemmer.

In der klinischen Praxis gehört Heparin zu den am häufigsten eingesetzten Antikoagulantien. Es katalysiert die Komplexbildung von Serinproteasen in der Gerinnungskaskade mit Antithrombin III. Seine antikoagulatorische Wirkung ist vorwiegend auf die Inhibition der Serinprotease Thrombin zurückzuführen.

Bei der Heparingabe besteht aber immer die Gefahr von Blutungskomplikationen, da eine komplette Thrombininhibition auch die vor Blutverlusten schützende extrinsische Gerinnung ausschaltet. Die Dosierung des Heparins muß deshalb anhand von Gerinnungstests in vitro genau eingestellt werden. Es handelt sich hier um einen schwierigen Balanceakt zwischen effektiver antithrombotischer Wirkung und dem Auftreten von Blutungskomplikationen.

Bei 1 - 5% der Patienten, die mit Heparin behandelt werden, kommt es zu einem Thrombozytenabfall.

Hirudin, ein Protein des Blutegels, ist der potenteste natürliche Thrombininhibitor. Es bildet mit Thrombin einen stöchiometrischen Komplex. Es gibt zur Zeit aber noch kein klinisch getestetes Antidot gegen Hirudin, so daß auch hier die Gefahr von Blutungskomplikationen nicht ausgeschlossen werden kann.

Weitere Antikoagulantien sind die Vitamin K-Antagonisten, die wie zum Beispiel Warfarin die Gammacarboxylierung verhindern und damit die Plasmakonzentration der aktiven Vitamin K-abhängigen Gerinnungsfaktoren II, VII, IX und X reduzieren. Der volle antikoagulatorische Effekt tritt aber erst 36-72 Stunden nach Gabe des Antagonisten ein. Sie eignen sich daher nicht zur sofortigen Behandlung akuter thrombotischer Krankheitszustände. Ebenso wie bei Heparin besteht die Gefahr von Blutungskomplikationen.

Thrombozytenaggregationshemmer, wie zum Beispiel Acetylsalicylsäure, sind die dritte Klasse der heute angewandten antithrombotischen Substanzen. Die Aktivierung der plasmatischen Gerinnungsfaktoren wird durch die Aggregationshemmer nicht inhibiert. Die Wirksamkeit beschränkt sich vorwiegend auf die Behandlung oder Prophylaxe arterieller Thrombosen.

Diese Beispiele zeigen, daß die heutige Behandlung thrombotischer Erkrankungen mit den zur Verfügung stehenden Präparaten einige entscheidende Nachteile mit sich bringt. Hauptsächlich sind die Ausschaltung der vor Blutverlusten schützenden extravaskulären Gerinnung und die Tatsache, daß die eingesetzten Präparate erst nach fast vollständigem Ablauf der Gerinnungskaskade eingreifen, als Nachteile zu nennen, die zu unkontrollierten Blutungskomplikationen führen können.

Generell ist es bei einer antithrombotischen Therapie wünschenswert, möglichst früh in das Gerinnungsgeschehen einzugreifen. Ist die Gerinnungskaskade erst einmal angestoßen, spielen sich teils irreversible Vorgänge ab (zum Beispiel Thrombozytenaktivierung) und die therapeutischen Interventionsmöglichkeiten beschränken sich dann auf eine Begrenzung des Schadens.

Der Erfindung lag die Aufgabe zu Grunde, eine Präparation zu finden, die einerseits die plasmatische Gerinnung inhibieren kann und gleichzeitig die vorhandenen Thrombozyten stabilisiert, so daß es zu keinem Abfall der Thrombozytenzahl kommt, und die andererseits die schützende extravaskuläre Gerinnung funktionstüchtig erhält.

Erfindungsgemäß wurde die Aufgabe durch die Bereitstellung von proteolytischen Fragmenten des F IX gelöst, die eine intakte gamma-Carboxyglutamat-Domäne (Gla) und Epidermal Growth Factor-like Domäne (EGF) enthalten und ein Molekulargewicht von ca. 12.000 bis 50.000 Dalton aufweisen.

Überraschenderweise wurde gefunden, daß die erfindungsgemäßen F IX-Fragmente in der Lage sind, den durch F IXa in vivo hervorgerufenen Abfall der Thrombozyten zu verhindern.

Dem F IX wird eine wichtige Rolle in der Hämostase zugesprochen, da sich der "extrinsic" und "intrinsic pathway" hier kreuzen (Mann K.G., Jenny R.J. et al, Annu Rev Biochem, 57, (1988), 915-956).

F IX ist ein Vitamin K-abhängiges Plasmaglykoprotein mit 416 Aminosäuren. Er enthält eine aminoständige Domäne mit gamma-Carboxy-glutaminsäure und zwei Domänen, die Sequenzen enthalten, die denen des Epidermal Growth Faktor (EGF) ähneln (Thompson A. R., Blood, 67, (1986), 565-572).6

Die Ergebnisse der bisherigen Untersuchungen der Wirkung und der Inhibition von F IX oder F IXa in verschiedenen Thrombosemodellen basierten auf der kompetetiven Hemmung der Besetzung der Endothelzellrezeptoren des F IXa. Bei den beschriebenen Gerinnungsvorgängen spielte die thrombozytenvermittelte Gerinnung nur eine untergeordnete Rolle.

In EP 0 263 529 wurde gezeigt, daß mit Peptiden, die die EGF-Domäne des F IX oder eine Untersequenz davon enthalten und ein Molekulargewicht von 500 - 1000 Dalton besitzen, die Thrombusbildung im Wesslerschen Venostase-Thrombose-Modell inhibiert werden kann. Grundlage hierfür war die Blockierung der Wechselwirkung des injizierten Faktor IXa mit Endothelzellen in einem isolierten Gefäßabschnitt durch die eine EGF-Sequenz enthaltenden Peptide.

Im Wessler-Tiermodell steht die Entstehung eines Thrombus im Vordergrund, der sich unter völligem Stillstand des Blutstromes (Stase) in dem isolierten Gefäßabschnitt bildet. Dies bedeutet, daß hier die thrombozytenvermittelte Gerinnungsaktivierung, wie sie im physiologischen Blutstrom abläuft, nur eine untergeordnete Rolle spielt.

Aussagen, inwieweit die EGF-Peptide Einfluß auf die physiologische thrombozytenvermittelte Gerinnung bei gleichzeitiger Abnahme der Thrombozytenzahl nehmen, sind nicht bekannt.

Zum Erreichen einer deutlich sichtbaren Inhibition der Thrombusbildung waren sehr hohe Peptidkonzentrationen notwendig. Das Massen-Verhältnis Peptid zu F IXa betrug ca. 1660 zu 1, das entspricht einem Molverhältnis von Peptid zu F IXa von 65000 zu 1.

Nachteilig sind bei der Verwendung von solch kleinen Peptiden auch die kurzen intravasalen Halbwertszeiten, die im Minutenbereich liegen.

Astermark und Stenflo, J. Biol. Chem., 260, 2438-2443, untersuchten verschiedene Domänen des Rinder-F IX, ihre Effekte auf die Blutgerinnung in vitro und ihre Wechselwirkung mit F IX-Rezeptoren an Rinderendothelzellen in vitro. Eine Wirkung der isolierten F IX-Domänen auf Thrombozyten wurde nicht beschrieben.

Mit der Anwendung eines durch Dansyl-Glu-Gly-Arg-(DEGR)-chloromethylketon im aktiven Zentrum chemisch inaktivierten F IXa konnte eine okklusive Thrombusbildung, ausgelöst durch elektrischen Strom, erfolgreich verhindert werden. Mit dem nativen F IX gelang es dagegen nicht, die Thrombusbildung zu inhibieren (Benedict, Ryan, et al., J. Clin. Invest., 88, (1991), 1760-1765).

Mit chemisch inaktiviertem F IXa konnte die Bildung von Fibringerinnseln im Tumorgewebe von Mäusen, denen Tumornekrosefaktor injiziert wurde, inhibiert werden. Mit dem Zymogen F IX war kein Unterschied zur Kontrolle festzustellen (Tijburg, Ryan, et al., J. Biol. Chem. 266, (1991), 12067-12074).

Andere Untersuchungen zeigten, daß die Endothelzellen und die Thrombozyten in der Gegenwart von F VIII und F X hochaffine Bindungsstellen für den F IXa, aber nicht für den F IX besitzen, die für den weiteren Ablauf der Gerinnung von Bedeutung sind (Stern, Nawroth, J. Biol. Chem. 260, 6717-6722; Hoffmann, Monroe, Throm. a. Hemost., 68, (1993), 74-78 und Ahmad, Rawala-Sheik J. Biol. Chem., 264, (1989), 20012-20016).

So konnte man aufgrund der bis heute gewonnenen Erkenntnisse über die Eigenschaften des F IX keinesfalls erwarten, daß bei der Verwendung der erfindungsgemäßen F IX-Fragmente eine Stabilisierung der Thrombozyten in vivo möglich ist.

Überraschenderweise wurde gefunden, daß durch die erfindungsgemäßen F IX-Fragmente eine Thrombozytenstabilisierung erfolgt, obwohl normalerweise eine massive Beteiligung der Thrombozyten an der intravasalen Fibrinbildung im physiologischen Blutstrom stattfindet, die dadurch zu einem Verbrauch der Thrombozyten führt.

### Herstellung der F IX-Fragmente:

Als Ausgangsmaterial für die Herstellung der erfindungsgemäßen F IX Fragmente können sowohl aus Plasma aufgereinigte F IX-Fraktionen als auch andere, z.B. gentechnologisch hergestellte F IX enthaltende Lösungen eingesetzt werden.

Erfindungsgemäß wird der Faktor IX mit Proteasen unter Bedingungen behandelt, bei denen die Gla- und mindestens eine EGF-Domäne weitestgehend intakt belassen werden, und bei denen Fragmente des F IX entstehen, die ein Molekulargewicht von rund 12.000 bis 50.000 D aufweisen. Dies kann z.B. durch spezifische Proteasen erreicht werden, die eine gleiche oder ähnliche Wirkungsweise wie z.B. Russell's Viper Venom aufweisen, wobei die erhaltenen Fragmente keine F IX-Aktivität mehr aufweisen und nicht chemisch modifiziert sein sollen.

Bei der Verwendung von Proteasen mit breiterer Spezifität, wie z.B. Chymotrypsin oder Trypsin, wird die proteolytische Spaltung unter Bedingungen durchgeführt, bei denen das aminoterminale Ende des F IX geschützt wird. Vorzugsweise wird der F IX mit seinem aminoterminalen Ende reversibel, das heißt nicht kovalent, an einen festen Träger gebunden und in der immobilisierten Form mit den Proteasen behandelt. Anschließend werden die antithrombotisch wirksamen Fragmente des F IX von dem Träger eluiert.

Als Träger können Anionenaustauscher oder Affinitätsträger für F IX, die den F IX am aminoterminalen Ende binden, eingesetzt werden. Besonders bevorzugt wird der F IX an alpha-Hydroxylaminopropyl-Gruppen tragende Polymere, wie z.B. Fractogel Amino-TSK, gebunden.

Eine weitere Möglichkeit der Herstellung der erfindungsgemäßen F IX-Fragmente ist die proteolytische Spaltung des F IX in Lösung mit Proteasen wie z.B. Chymotrypsin oder Trypsin, die vorzugsweise an einen Träger immobilisiert sind, wobei das aminoterminale Ende des F IX mit niedermolekularen Substanzen, die an die Gla- und EGF-Domäne binden, geschützt wird. Als solche Schutzsubstanzen werden vorzugsweise niedermolekulare Amine und besonders bevorzugt 1-Amino-2-propanol verwendet. Denkbar ist aber auch der Einsatz von anderen Schutzsubstanzen, wie z.B. Protamin.

Die proteolytischen Spaltprodukte des F IX können gegebenenfalls mit milden Reduktionsmitteln, wie z.B. Dithiothreitol, unter schonenden Bedingungen behandelt werden. Auf diese Weise läßt sich z.B. prinzipiell aus F IXa ein inhibitorisch wirksames, chemisch nicht kovalent modifiziertes Fragment ohne F IX-Aktivität isolieren.

Es ist bekannt, daß bei der herkömmlichen Behandlung des F IX mit alpha-Chymotrypsin entweder Spaltprodukte entstehen, die keine intakten Gla- oder EGF-Domänen enthalten oder die Ausbeute an Spaltprodukten mit Gla- und EGF-Domänen nur sehr gering ist (Wildgoose et al., Biochem. and Biophys. Res. Comm., 152 (1988), 1207-1212.). Auf jeden Fall stellte die kontrollierte Durchführung der proteolytischen Spaltung bisher ein Problem dar, da immer ein Gemisch verschiedener Spaltprodukte entstand.

Demgegenüber gestattet es das erfindungsgemäße Verfahren erstmals, die wirksamen Fragmente technisch reproduzierbar in hohen Ausbeuten rein zu gewinnen.

Die Vorteile der erfindungsgemäßen Herstellungsverfahren sind unter anderem:
1. Ein Verzicht auf hochtoxische Proteaseinhibitoren (z.B.Diisopropylfluorophosphat) für die Inaktivierung der für die Spaltung verwendeten Proteasen.
2. Ein Verzicht auf eine chemische Modifizierung der F IX-Spaltprodukte.
3. Eine schonende proteolytische Spaltung des F IX, bedingt durch die Fixierung seines aminoterminalen Endes an eine Matrix, vorzugsweise eine 2-Hydroxyl-aminopropyl-Gruppen tragende Matrix.
4. Eine schonende proteolytische Spaltung des F IX durch das Einsetzen von niedermolekularen Aminen zum Schutz der am aminoterminalen Ende befindlichen Domänen.
5. Eine chromatographische Trennung der antithrombotisch wirksamen F IX-Fragmente von unwirksamen Fragmenten.

### Beispiel 1:

### Herstellung von F IX-Spaltprodukten aus nativem F IX, der während der Proteolyse durch alpha-Chymotrypsin an Fractogel Amino TSK gebunden ist.

Eine 50 ml Säule mit Fractogel Amino-TSK (Merck, Darmstadt), einem Copolymerisat von Glycidylmethacrylat, Pentaerythroldimethacrylat und Polyvinylalkohol an das alpha-Hydroxylaminopropyl-gruppen gebunden sind, wird mit Tris-HCl-Puffer (0,05 M Tris-HCl, 0,05 M NaCl, pH 7,3) äquilibriert. 16 mg F IX mit einer spezifischen Aktivität von 110 U/mg, gelöst im Tris-HCl-Puffer, werden auf die Säule aufgetragen. Anschließend wird die Säule mit dem gleichen Puffer, dem noch 10 mM Kalziumchlorid zugesetzt wurden, gewaschen.

Zur Fragmentierung des gebundenen F IX wird nun mit Chymotrypsin, gelöst in dem Kalzium enthaltenden Tris-HCL-Puffer, inkubiert. Die Konzentration der Protease in dem Puffer beträgt 0,025 mg/ml. Man spült die Säule mit diesem Puffer bei einer Flußgeschwindigkeit von 100 ml/h und erhält nach einem Säulenvolumen Eluat eine Proteinfraktion, die aus Chymotrypsin und Spaltprodukten des F IX besteht, die unter den gewählten Bedingungen keine Affinität gegenüber der Fractogel Amino-TSK-Säule besitzen. Diese mit der Protease verunreinigte Fraktion wird verworfen.

Nach dem Spülen mit 2,5 Säulenvolumen des Tris-HCl-Chymotrypsinpuffers wird mit Tris-HCl-Puffer ohne Protease und Kalzium gewaschen, bis kein Protein mehr von der Säule eluiert wird.

In einem weiteren Schritt wird die Matrix mit Tris-HCl-Puffer (0,05 M Tris-HCl, 0,15 M NaCl, pH 7,3) gespült, bis kein Protein mehr eluiert wird.

Zur Elution der wirksamen F IX-Spaltprodukte erhöht man die NaCl-Konzentration des Tris HCl-Puffers auf 0,5 M. Die gewünschte Fraktion wird mit einem Volumen von 70 ml eluiert. Die Fragmente besitzen keine F IX-Aktivität mehr.

In vitro inhibieren die Fragmente die F X-Aktivierung in Gegenwart von Endothelzellen sowie die Gerinnung von F IXa enthaltendem Plasma unter Beteiligung von Phospholipiden. Dagegen inhibieren sie nicht die Gerinnung einer Plasmaprobe, der Thrombin zugesetzt wurde.

Die F IX-Fragmente werden gegen 75 mM NaCl dialysiert und anschließend lyophylisiert.

Bei einer SDS-Gelelektrophorese der Fragmentfraktion unter nicht reduzierenden Bedingungen sind zwei Banden mit einem Molekulargewicht von etwa 47 000 und 34 000 Dalton zu erkennen, die dem aminoterminalen Ende des F IX zugeordnet werden können.

Die Ausbeute an wirksamen F IX-Spaltprodukten, bezogen auf die eingesetzte Proteinmenge an F IX, lag bei 60 %.

### Beispiel 2:

### Herstellung von F IX-Spaltprodukten aus nativem F IX, der während der Proteolyse durch Trypsin an Fractogel Amino TSK gebunden ist.

10 mg F IX wurden analog Beispiel 1 behandelt mit dem Unterschied, daß an Stelle des Chymotrypsins Trypsin in einer Konzentration von 0,05 mg/ml eingesetzt wurde.

In der SDS-Gelelektrophorese unter reduzierenden Bedingungen ist eine Hauptbande bei etwa 33 000 Dalton zu sehen, die dem aminoterminalen Ende des F IX zugeordnet werden kann.

Die Ausbeute an wirksamen F IX-Fragmenten, bezogen auf die eingesetzte F IX-Menge, beträgt in diesem Ansatz 57 %.

Die F IX-Fragmente besitzen keine F IX-Aktivität mehr. Sie hemmen in vitro die F X-Aktivierung gegenüber Endothelzellen und die Gerinnung von F IXa enthaltenden Plasma unter Beteiligung von Phospholipiden. Die Gerinnung einer mit Thrombin versetzten Plasmaprobe wird nicht inhibiert.

### Beispiel 3:

### Herstellung von F IX-Spaltprodukten unter Verwendung von 1-Amino-2-propanol.

Zu 16 mg F IX mit einer spezifischen Aktivität von 110 U/mg, gelöst in 60 ml Tris HCL-Puffer (0,05 M Tris-HCl, 0,15 M NaCl, pH 7,5), werden 10 mM 1-Amino 2-Propanol hinzugegeben. Diese Lösung wird nun mit einer Flußrate von 200 ml/h über eine 15 ml Säule mit alpha-Chymotrypsin-Sepharose gepumpt. In Abständen von 15 Minuten wird die verbliebene F IX-Aktivität in der Lösung mit dem Einphasentest bestimmt. Ist keine F IX-Aktivität mehr in der Lösung nachweisbar, wird die Säule mit Puffer gewaschen, bis kein Protein mehr von der Säule eluiert.

Es folgt eine Dialyse der Lösung mit den F IX-Spaltprodukten gegen Tris HCl-Puffer (0,05 M Tris HCl, 0,2 M NaCl, pH 7).

Eine Aufreinigung der erwünschten F IX-Fragmente erreicht man über eine Chromatographie mit einer 2-Hydroxyl-amino-Gruppen tragende Matrix, dem Fractogel Amino-TSK.

Eine 50 ml Säule mit Fractogel Amino-TSK wird mit Tris-HCl-Puffer (50 mM Tris-HCl, 0,1 M NaCl, pH 7) äquilibriert. Die Lösung mit den F IX-Spaltprodukten wird mit aqua dest 1 zu 2 verdünnt und mit einer Flußgeschwindigkeit von 300 ml/h auf die Säule gegeben. Die antithrombotisch wirksamen F IX-Spaltprodukte binden an die Säule, während die unwirksamen und andere Verunreinigungen durchlaufen.

Nach einem Waschschritt mit Tris-HCl-Puffer (0,05 M Tris-HCl, 0,15 M NaCl, pH 7) werden die antithrombotisch wirksamen F IX-Spaltprodukte mit Tris-HCl-Puffer( 0,05 M Tris-HCl, 0,5 M NaCl, pH 7) in einem Volumen von 40-60 ml eluiert. Sie besitzen die gleichen Eigenschaften wie in Beispiel 1 beschrieben. Ihr Molekulargewicht liegt bei etwa 50 000 Dalton und sie können dem aminoterminalen Ende des F IX zugeordnet werden.

Nach erneuter Dialyse gegen 0,05 M Tris-HCl, 75 mM NaCl, pH 7 werden die F IX-Fragmente lyophilisiert. Die Ausbeute, bezogen auf die eingesetzte Proteinmenge an F IX, betrug 63 %.

### Beispiel 4:

### Herstellung von F IXa-Spaltprodukten aus aktiviertem F IX, der während der Proteolyse an Fractogel Amino TSK gebunden ist.

Russel-Viper Venom (RVV, Fa. Sigma, Heidelberg) wird an eine CNBr-aktivierte Sepharose 4 B nach der Vorschrift des Herstellers (Pharmacia, Schweden) gebunden. 1 ml Gel enthält 1,5 mg RVV. Eine F IX-Lösung mit einer spezifischen Aktivität von 110 U/mg wird über diese Säule gepumpt, bis keine Aktivitätserhöhung mehr festzustellen ist. Die Lösung wird gegen Tris-HCL-Puffer (0,05 M Tris-HCL, 0,05 M NaCl, pH 7,3) dialysiert.

Eine 30 ml Säule mit Fractogel Amino-TSK wird mit dem Tris-HCl-Puffer äquilibriert. Anschließend wird die aktivierte F IX-Lösung auf die Säule aufgetragen. Dann wird mit dem gleichen Puffer, dem noch 10 mM Kalziumchlorid zugesetzt wurden, gewaschen.

Zu dem Puffer mit Kalziumchlorid wird nun alpha-Chymotrypsin in einer Konzentration von 0,015 mg/ml hinzugesetzt und diese Lösung im Kreislauf mit einer Flußgeschwindigkeit von 75 ml/h über die Säule gegeben. Man erhält nach einem Säulenvolumen Eluat eine Fraktion, die aus alpha-Chymotrypsin und Spaltprodukten des F IXa besteht, welche keine Affinität zu der Amino TSK besitzen.

Nach dem Spülen mit 3 Säulenvolumen des Chymotrypsin-haltigen Puffers wird mit Tris-HCl-Puffer ohne Protease gewaschen, bis kein Protein mehr von der Säule eluiert wird.

Die Matrix wird mit 0,05 M Tris-HCL, 0,15 M NaCl, pH 7 gespült, bis kein Protein mehr eluiert wird und anschließend mit 0,05 M Tris-HCL, 0,5 M NaCl, pH 7 eluiert.

Die Ausbeute an wirksamen F IXa-Fragmenten lag, bezogen auf die eingesetzte Menge des F IXa, bei 60 %.

Eine SDS-Gelelektrophorese unter nicht reduzierenden Bedingungen zeigte eine Hauptbande bei einem Molekulargewicht von etwa 31 000 Dalton, die dem aminoterminalen Ende des F IX zugeordnet werden kann.

Die F IXa-Spaltprodukte werden gegen 75 mM NaCl dialysiert und lyophilisiert.

Die Fragmente besitzen keine F IXa-Aktivität mehr und zeigen in vitro eine Inhibition der F X-Aktivierung in Gegenwart von Endothelzellen und der Gerinnung von F IXa-enthaltenden Plasma unter Beteiligung von Phospholipiden. Dagegen inhibieren sie nicht die Gerinnung einer Plasmaprobe, der Thrombin zugesetzt wurde.

Überraschenderweise konnte mit den erfindungsgemäßen F IX-Fragmenten in vivo eine Inhibition des durch die Gabe von F IXa ausgelösten drastischen Thrombozytenabfalles erreicht werden. F IXa ist ein starkes Thrombogen, daß bei Infusion in Kaninchen intravasale Thrombosen auslöst.

### Tierexperimentelle Studien am Kaninchen

5 narkotisierten Kaninchen injizierte man über eine Kanüle in einer Ohrrandvene 0,12 mg/kg Körpergewicht F IXa.

Weiteren 5 Tieren wurde 5-10 Minuten vor Gabe des F IXa eine Dosis von 1,8 mg/kg Körpergewicht F IX-Fragmente aus Beispiel 1 über die Ohrrandvene injiziert.

5 Tiere erhielten als Kontrolle statt F IXa 0,2 ml/kg Körpergewicht physiologische NaCl-Lösung injiziert.

Über die gesamte Versuchsdauer von 60 Minuten wurden der arterielle Blutdruck, die Atemfrequenz und das Standard-EKG registriert und zu bestimmten Zeitpunkten arterielles Blut in ein Citratrörchen abgenommen. In dem abgenommenen Blut wurden die Thrombozytenzahl und bei einigen Tieren die APTT und Thrombinzeit bestimmt.

Außer bei der Präparation der Kaninchen wurde das Gefäßsystem der Tiere nicht beeinträchtigt, so daß ein physiologischer Blutstrom in den Gefäßen gewährleistet war.

In der Kontrolltiergruppe konnte man über den gesamten Versuchsablauf einen leichten Abfall der Thrombozytenzahl erkennen. Dies ist ein Hinweis darauf, daß allein die Präparation der Tiere und die Narkose Auswirkungen auf die Thrombozytenstabilität besitzen.

Bei Tieren, die F IXa erhielten, konnte aber im Vergleich zur Kontrollgruppe mit NaCl-Lösung ein drastischer, signifikanter Abfall der Thrombozytenzahlen festgestellt werden. Die Tiere zeigten Symptome einer disseminierten intravasalen Gerinnung (DIC). Starker Blutdruckabfall sowie starke Schwankungen in der Atemfrequenz und im EKG traten auf.

Sehr deutliche Hinweise auf die Gerinnungsaktivierung waren das Gerinnen einiger Blutproben in den Citratröhrchen und die Verkürzung der APTT beziehungsweise die Verlängerung der Thrombinzeit, was auf den Verbrauch von Fibrinogen und die Entstehung von Fibringerinnseln in den Gefäßen hindeutet.

Die Kaninchen, die 5- 10 Minuten vor der F IXa-Gabe die erfindungsgemäßen F IX-Fragmente erhielten, zeigten keine der oben beschriebenen Unverträglichkeitsreaktionen. Die Thrombozytenzahl blieb überraschenderweise im Bereich der Kontrolltiergruppe. Ein Gerinnen der abgenommenen Blutproben blieb in jedem Fall aus. Die APTT und die Thrombinzeit blieben relativ konstant. Dies bedeutet, daß eine Gerinnungsaktivierung verhindert wurde, ohne die schützende extravaskuläre Gerinnung auszuschalten.

Die alleinige Gabe von 2 mg/kg Körpergewicht F IX-Fragmenten zeigte keinerlei Unverträglichkeitsreaktionen und bewirkten, daß die Thrombozytenzahlen über dem Niveau der Kontrolltiergruppe lagen. Die Auswirkungen der Präparation der Tiere und deren Narkose auf die Thrombozytenstabilität wurde durch die F IX-Fragmente verhindert.

Mit den erfindungsgemäßen F IX-Spaltprodukten ist es möglich, eine massive thrombozytenvermittelte Gerinnungsaktivierung in vivo zu verhindern und gleichzeitig die Thrombozyten zu stabilisieren. Die schützende extravasale Gerinnung wird durch die Behandlung nicht beeinträchtigt.

## Patentansprüche

1. Wirkstoff mit antithrombotischer Wirkung auf der Basis von proteolytischen Spaltprodukten des humanen Blutgerinnungsfaktors IX, dadurch gekennzeichnet, daß die Faktor IX-Fragmente
a.) die Gla-Domänen und mindestens eine EGF-Domäne enthalten,
b.) keine Faktor IX-Gerinnungsaktivität zeigen,
c.) chemisch nicht modifiziert sind und
c.) ein Molekulargewicht von ca. 12 000 bis 50 000 Dalton aufweisen.

2. Arzneimittel zur Prophylaxe und Behandlung thrombotischer Erkrankungen, enthaltend oder bestehend aus dem Wirkstoff nach Anspruch 1.

3. Verfahren zur Herstellung eines Wirkstoffs nach Anspruch 1, dadurch gekennzeichnet, daß man F IX an einen Träger, der F IX am aminoterminalen Ende bindet, bindet, proteolytisch spaltet, die Spaltprodukte vom Träger löst, aufreinigt und gegebenenfalls lyophilisiert.

4. Verfahren nach Anspruch 3, dadurch gekennzeichnet, daß man als festen Träger einen Anionenaustauscher oder einen Affinitätsträger mit Affinität zum aminoterminalen Ende des F IX verwendet.

5. Verfahren nach Anspruch 3 und 4, dadurch gekennzeichnet, daß der feste Träger alpha-Hydroxyl-amino-propyl-Gruppen als funktionelle Gruppen trägt.

6. Verfahren nach Anspruch 3 und 4, dadurch gekennzeichnet, daß der feste Träger ein Copolymerisat von Glycidylmethacrylat, Pentaerythroldimethacrylat und Polyvinylalkohol mit alpha-Hydroxyl-amino-propyl-Gruppen ist.

7. Verfahren zur Herstellung eines Wirkstoffs nach Anspruch 1, dadurch gekennzeichnet, daß man den Faktor IX in Lösung unter Zugabe von Schutzsubstanzen proteolytisch spaltet, die Spaltprodukte abtrennt, aufreinigt und gegebenenfalls lyophilisiert.

8. Verfahren nach Anspruch 7, dadurch gekennzeichnet, daß man als Schutzsubstanz niedermolekulare Amine verwendet.

9. Verfahren nach Anspruch 7 und 8, dadurch gekennzeichnet, daß man den Faktor IX in Lösung unter Zugabe von 1-Amino-2-propanol proteolytisch spaltet.

10. Verfahren nach Anspruch 7 bis 9, dadurch gekennzeichnet, daß die Protease an einem festen Träger immobilisiert ist.

11. Verfahren nach Anspruch 3 bis 10, dadurch gekennzeichnet, daß man den Faktor IX vor der proteolytischen Spaltung zu Faktor IXa aktiviert.

12. Verfahren nach Anspruch 3 bis 11, dadurch gekennzeichnet, daß man als Protease alpha-Chymotrypsin verwendet.

13. Verfahren nach Anspruch 3 bis 11, dadurch gekennzeichnet, daß man als Protease Trypsin verwendet.

14. Verfahren nach Anspruch 3 bis 13, dadurch gekennzeichnet, daß man als Ausgangsmaterial Faktor IX, der aus humanem Plasma oder Faktor IX-haltigen Plasmafraktionen oder aus Zellkultur-Medien gewonnen wurde, verwendet.

15. Verwendung des Wirkstoffs nach Anspruch 1 zur Herstellung eines Arzneimittels zur Prophylaxe und Behandlung thrombotischer Erkrankungen.
